# EUROPÄISCHE PATENTANMELDUNG

(11) **EP 4 345 162 A1**
(43) Veröffentlichungstag der Anmeldung: **03.04.2024**
(21) Anmeldenummer: 22198048.5
(22) Anmeldetag: 27.09.2022
(51) Int. Cl.: C12N 15/10, G01N 1/40

(54) **PARTIKEL MIT OLIGOMEREN UND/ODER POLYMEREN URONSÄUREN ZUR ANREICHERUNG VON BIOLOGISCHEM MATERIAL**

(71) Anmelder: LGC Genomics GmbH, 12459 Berlin (DE)
(72) Erfinder: Schubert, Frank, 12623 Berlin (DE); Hauser, Heiko, 10437 Berlin (DE); Rogalla, Tanita, 12439 Berlin (DE); von der Heide, Eva Kristin, 12053 Berlin (DE); Hoffmann, Melanie, 14641 Nauen (DE); Brinckmann, Anja, 12161 Berlin (DE)
(74) Vertreter: Gulde & Partner

(57) **Zusammenfassung**

Die Erfindung betrifft Partikel zur Anreicherung von biologischem Material, wobei oligomere und/oder polymere Uronsäuren an die Partikel gebunden sind. Ferner betrifft die Erfindung die Verwendung der Partikel zur Anreicherung von biologischem Material sowie ein Verfahren zur Herstellung der Partikel. Die Erfindung weist insbesondere dahingehend Vorteile auf, dass die Anreicherung von biologischem Material im neutralen oder schwach saurem Milieu erfolgen kann als auch dahingehend, dass auf die Zugabe von zwei- oder mehrwertigen Metallkationen verzichtet wird.

## Beschreibung

Die Erfindung betrifft Partikel zur Anreicherung von biologischem Material, wobei oligomere und/oder polymere Uronsäuren an die Partikel gebunden sind. Ferner betrifft die Erfindung die Verwendung der Partikel zur Anreicherung von biologischem Material sowie Verfahren zur Herstellung der Partikel.

Seit einiger Zeit liefert die Analyse sogenannter Biomarker wertvolle Hinweise auf die Zusammensetzung von biologischen Proben. Hierdurch kann beispielswiese der Gesundheitszustand eines untersuchten Organismus festgestellt werden. Diese Biomarker können zum einen Stoffwechselprodukte sein, aber auch Pathogene wie Bakterien oder Viren.

Insbesondere spielen für mehrere diagnostische Zielstellungen sogenannte zellfreie zirkulierende DNAs eine besondere Rolle. Für diese diagnostischen Prozesse ist es heutzutage von besonderer Bedeutung, dass diese Prozesse standardisiert und automatisiert erfolgen können, was die parallele Analyse vieler Proben im Verlauf eines einzelnen Analysenganges erlaubt.

Nachteilig kann bei einer solchen Analyse allerdings sein, dass die betreffenden Biomarker in sehr geringer Konzentration in der Probe vorhanden sind und deshalb oft große Probenvolumina in den Analysengang eingebracht werden müssen. Das erschwert die notwendige Automatisierung von Hochdurchsatzprozessen in der diagnostischen Routine.

Um diese Probleme zu überwinden, wurden bislang physikalische Verfahren, insbesondere Zentrifugation- und Filtrationsverfahren zur Anreicherung von biologischem Material genutzt.

Die Nutzung dieser physikalischen Verfahren ist jedoch kostenintensiv (hoher Investitionsaufwand für Spezialgeräte, insbesondere Zentrifugen) und langwierig.

Es gibt deshalb seit langer Zeit Bestrebungen, die Biomarker vor dem eigentlichen Analysengang auf alternative Art und Weise anzureichern und das Arbeitsvolumen im Analysengang zu verringern.

Zum Beispiel wird in DE 102015215894 ein Verfahren beschrieben, in dem Lösungen von Alginsäuren im Probenmaterial unter Zugabe von mehrwertigen Kationen in saurem Medium geliert werden und dabei biologische Materialien einschließen.

Nach Abtrennen der Gelpartikel befinden sich die biologischen Materialien in einem kleinen Pellet, der Überstand kann verworfen und das Pellet in einem sehr kleinen Volumen analysiert werden.

DE 102015215894 beschreibt ein Verfahren, bei welchem eine Gelbildung in Gegenwart von magnetischen Partikeln erfolgt und damit die Abtrennung des gebildeten Gels mittels externer magnetischer Felder erfolgen kann. Allerdings sind auch in dieser Variante zur Bildung des Gels, welches im Stande ist, die Bindung des biologischen Materials zu vollziehen, nachteiligerweise zwei- oder mehrwertige Kationen als auch ein stark saurer pH-Wert erforderlich.

Der Erfindung liegt nun die Aufgabe zugrunde eine Möglichkeit zur Anreicherung von biologischem Material bereitzustellen, welche die aus dem Stand der Technik bekannten Probleme überwindet.

Gelöst wird diese Aufgabe durch Partikel zur Anreicherung von biologischem Material, wobei oligomere und/oder polymere Uronsäuren an die Partikel gebunden sind. Gemäß weiteren Aspekten betrifft die Erfindung die Verwendung der Partikel zur Anreicherung von biologischem Material sowie Verfahren zur Herstellung der Partikel.

Die vorliegende Erfindung grenzt sich damit qualitativ von den bekannten Lösungen aus dem Stand der Technik ab. Zwar offenbart der Stand der Technik die Verwendung von Partikeln zur Anreicherung von Nukleinsäuren. Allerdings findet, anders als im Falle der vorliegenden Erfindung, eine Gelierung der Uronsäuren durch Mineralsäure und/oder die Verwendung mehrwertiger Metallkationen statt. Die hierdurch gebildeten Hydrogele schließen sowohl die Nukleinsäuren als auch die magnetischen Partikel ein. Die magnetischen Partikel dienen in dem Verfahren gemäß dem Stand der Technik also nicht zur (direkten) Bindung der Nukleinsäuren, sondern lediglich zur späteren Abtrennung des Pellets. Das erfindungsgemäße Verfahren hingegen setzt auf eine (kovalente) Bindung der Uronsäuren an das Trägermaterial der Partikel. Die Funktionalisierung der Partikel mit den Uronsäuren findet daher bevorzugt bereits vor der Verwendung der Partikel zur Anreicherung des biologischen Materials statt.

Es wurde überaschenderweise festgestellt, dass es eine Affinität von biologischem Material zu immobilisierten, an feste Oberflächen gebundenen, nativen, oligomeren und polymeren Uronsäuren gibt. Wurden diese nativen Uronsäuren vorzugsweise kovalent an indifferenten Oberflächen fixiert, dann war es möglich aus Proben, die biologisches Material enthalten, dieses biologische Material an die mit Uronsäuren modifizierten Oberflächen zu binden und aus dem Probenmaterial zu entfernen. Es war überraschender Weise nicht erforderlich zusätzliche Komponenten, insbesondere zwei- oder mehrwertige Metallkationen, in die Probenlösung zu geben, um die Uronsäuren zu gelieren.

Indifferente Oberflächen in Sinne der vorliegenden Erfindung sind funktionalisierbare Oberflächen, welche bevorzugt keine ausgeprägte spezifische Affinität gegenüber dem zu isolierenden biologischem Material haben, das zu isolierende biologische Material also, wenn überhaupt, nur in geringem Umfang und nicht in analytisch relevanten Mengen adsorbieren.

Das Verfahren ist geeignet biologisches Material aus großen Volumina anzureichern und damit die Sensitivität von diagnostischen Untersuchungen zu erhöhen. Außerdem gestattet es das Arbeitsvolumen in einem Analysengang deutlich zu verringern und damit den gesamten Analysenprozess automatisierungsfreundlich zu gestalten.

Die Erfindung weist insbesondere dahingehend Vorteile auf, dass die Anreicherung von biologischem Material im nicht sauren oder allenfalls schwach sauren Milieu erfolgen kann als auch dahingehend, dass auf die Zugabe von zwei- oder mehrwertigen Metallkationen, wie sie im bekannten Stand der Technik erforderlich ist und/oder die Zugabe von Mineralsäure verzichtet werden kann. Bevorzugt erfolgt die Anreicherung bei einem pH Wert von nicht unter 5, weiter bevorzugt bei einem pH Wert von nicht unter 6, noch weiter bevorzugt bei einem pH Wert von nicht über 7. Besonders bevorzugt wird die Anreicherung im neutralen Milieu. durchgeführt.

Gemäß einer bevorzugten Ausführungsform werden die Partikel beschrieben, wobei die oligomeren und/oder polymeren Uronsäuren lineare 1-4 glycosidische verknüpfte Einheiten aus α-1,4-L-Guluronsäure und/oder aus β-1,4-D-Mannuronsäure aufweisen.

Als oligomere und polymere Uronsäuren kommen also bevorzugt Alginate insbesondere lineare 1-4 glycosidisch verknüpfte β-D-Mannuronsäuren und/oder α-L-Guluronsäuren sowie Copolymere beider Uronsäuren zum Einsatz.

Gemäß einer bevorzugten Ausführungsform werden die Partikel beschrieben, wobei die oligomeren und/oder polymeren Uronsäuren Alginsäuren umfassen. Das Alginat weist bevorzugt eine molekulare Masse von 120,000-190,000 g/moL und weiter bevorzugt ein Verhältnis von Mannuronsäure zu Guluronsäure (M/G Verhältnis) von 1 bis 2 auf weiter bevorzugt von 1.3 bis 1.6.

Besonders bewährt haben sich native Alginsäuren aus unterschiedlichen Braunalgen, die im großen Umfang und unterschiedlicher Zusammensetzung erhältlich sind.

Als Algengattungen für die kommerzielle Herstellung von Alginat dienen unter anderem Laminaria, Ecklonia, Macrocystis, Lessonia, Ascophylum und Durvillea.

Ein besonderes Merkmal der Erfindung ist, dass die verwendeten Materialien keinen zusätzlichen Einsatz von aggressiven und/oder umweltschädlichen Reagenzien erfordern. Die Materialien selbst sind nativen Ursprungs sowie nicht ökologisch bedenklich.

Gemäß einer bevorzugten Ausführungsform werden die Partikel beschrieben, wobei das biologische Material Viren, Bakterien, Apikomplexa, Proteine und insbesondere Nukleinsäuren umfasst.

Insbesondere ist die vorliegende Erfindung zur Anreicherung und zum Nachweis von zirkulierender freier DNA (cfDNA) geeignet.

Zirkulierende freie DNA (cfDNA) sind abgebaute DNA-Fragmente (50 - 200 bp), die an das Blutplasma abgegeben werden. cfDNA kann verwendet werden, um verschiedene Formen von DNA zu beschreiben, die frei im Blutstrom zirkulieren, einschließlich zirkulierender Tumor-DNA (ctDNA), zellfreier mitochondrialer DNA (ccf mtDNA) und zellfreier fötaler DNA (cffDNA). Erhöhte cfDNA-Spiegel werden bei Krebs beobachtet, insbesondere bei fortgeschrittener Erkrankung. Es gibt Hinweise darauf, dass cfDNA mit zunehmendem Alter immer häufiger im Blutumlauf anzufinden ist. Es hat sich gezeigt, dass cfDNA ein nützlicher Biomarker für eine Vielzahl von Krankheiten ist. Zellfreie DNA (cfDNA) ist im zirkulierenden Plasma und in anderen Körperflüssigkeiten vorhanden. Die Freisetzung von cfDNA in den Blutkreislauf erfolgt aus verschiedenen Zellen darunter Primärtumore, Tumorzellen, die im peripheren Blut zirkulieren, metastatischen Ablagerungen an entfernten Stellen und auch aus normalen Zelltypen, wie hämatopoetische und stromale Zellen. Tumorzellen und cfDNA zirkulieren im Blutkreislauf von Krebspatienten. Die rasch erhöhte Akkumulation im Blut während der Tumorentwicklung wird durch eine übermäßige DNA-Freisetzung durch apoptotische Zellen und nekrotische Zellen verursacht. Aktive Sekretion innerhalb von Exosomen wird ebenfalls diskutiert. cfDNA zirkuliert überwiegend als Nukleosomen, die Kernkomplexe aus Histonen und DNA sind. cfDNA kann auch in kürzeren Größenbereichen (z. B. 50 bp) beobachtet und mit regulatorischen Elementen assoziiert sein. Diese sind bei Krebs häufig unspezifisch erhöht, können aber für die Überwachung einer zytotoxischen Krebstherapie spezifischer sein, hauptsächlich für die frühzeitige Abschätzung der Therapiewirksamkeit.

Insbesondere ist die vorliegende Erfindung ferner zur Anreicherung und zum Nachweis von viralen Partikel geeignet, beispielsweise pathogenen RNA-Viren.

Gemäß einer bevorzugten Ausführungsform werden die Partikel beschrieben, wobei die oligomeren und/oder polymeren Uronsäuren mittels eines molekularen Linkers (L) an die Partikel gebunden sind.

Beispielhaft soll hier das Einführen von Aminogruppen durch Reaktion silikatischer Materialien mit Aminopropyl-triethoxy-silan angeführt werden. Andere Verfahren, wie etwa der Umsatz des indifferenten Trägermaterials mit Trichlor-triazin bzw. dessen Derivaten sind ebenfalls möglich. Dem Fachmann sind die umfangreichen Möglichkeiten zur Einführung von Ankergruppen/molekularen Linkern in feste Oberflächen von sowohl organischen als auch anorganischen Basismaterialien bekannt ("Enzyme immobilization: an overview on techniques and support materials", Biotech. 2013 Feb; 3(1): 1-9).

Die kovalente Fixierung der oligomeren bzw. polymeren Uronsäuren kann sowohl über die in diesen Strukturen enthaltenen Carboxylgruppen, als auch über die Hydroxylgruppen erfolgen. Eine weitere Möglichkeit besteht in der Modifikation der verwendeten Uronsäuren um in die nativen Strukturen zusätzliche Funktionalitäten für eine kovalente Fixierung an Oberflächen einzuführen. Bekannte Möglichkeiten für diese Vorgehensweise sind beispielhaft in Rosiak et al. beschrieben (Rosiak, Piotr; Latanska, Ilona; Paul, Paulina; Sujka, Witold and Kolesinska, Beata; Modificaion of Alginates to Modulate Their Physic-Chemical Properties and Obtain Biomaterials with Different Functional Properties Molecules 2021, 26,7264).

Bekannt sind solche Kupplungsreaktionen dem Fachmann auch aus der Peptidsynthese. Bevorzugt findet eine Bindung mittels eines molekularen Linkers (L) an die Partikel durch eine Kupplungsreaktion statt.

Durch die Verwendung eines Kopplungsreagenz wird die Polyuronsäure über die Carboxylgruppe vorübergehend in eine reaktive Zwischenstufe überführt, die anschließend bereitwillig mit einem Nukleophil reagiert. Grundsätzlich können eine Vielzahl an Kupplungsreagenzien verwendet werden. Da nicht jede Kombination der aktivierten Partikel mit einem Kupplungsreagenz die gleiche Ausbeute aufweist, sind mache Verfahren beziehungsweise Reagenzien bevorzugt. Wasserlösliche Kupplungsreagenzien können zur Immobilisierung der Polyuronsäuren in wässrigen Lösungen verwendet werden. Kupplungsreagenzien können in verschiedene Stoffklassen unterteilt werden.

Gemäß einer bevorzugten Ausführungsform werden die Partikel beschrieben, wobei der molekulare Linker (L) von einer Aminopropylkette gebildet wird.

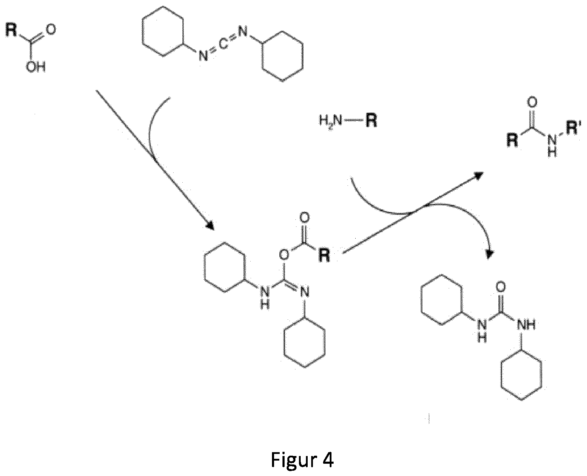

Dieser molekulare Linker wird exemplarisch mit den durch Carbodiimid aktivierte Carboxylgruppen der Alginsäure umgesetzt. Die Reaktion ist schematisch in Figure 4 dargestellt.

Gemäß einer bevorzugten Ausführungsform werden die Partikel beschrieben, wobei die Partikel ein anorganisches Basismaterial umfassen.

Allerdings sind neben anorganischen Materialien grundsätzlich auch organische Materialien denkbar. Dabei sollten diese Materialien dahingehend ausgestaltet sein, dass diese bevorzugt eine indifferente Oberfläche bilden.

Es kommen dabei bevorzugt anorganische Materialien wie Glasfasern oder poröse Gläser (CPG) zum Einsatz, die mit bekannten Verfahren mit funktionellen Gruppen modifiziert sind und eine kovalente Immobilisierung von oligomeren oder polymeren Uronsäuren erlauben. Besonders bevorzugt umfasst das anorganische Basismaterial Siliziumoxid, insbesondere in Form von Silicagel.

Dem Fachmann ist bewusst, dass sich alle indifferenten Materialien, die mit entsprechenden zur Anbindung von Alginsäuren verwendbaren funktionellen Gruppen modifizieren lassen in Sinne der Erfindung verwenden werden können. So können auch beispielsweise Mikropartikel aus organischen Basismaterialien wie Polystyren, Polymethacrylat, Melamin und ähnlichem im Sinne der Erfindung verwenden lassen, sofern sie sich gegenüber dem anzureichernden biologischen Material indifferent im Sinne dieser Erfindung verhalten und über entsprechende Möglichkeiten zur Modifikation mit Linkergruppierungen verfügen. Auf entsprechende Literatur ist bereits weiter oben hingewiesen worden.

Ganz besonders als feste Oberflächen geeignet sind magnetische Mikropartikel insbesondere aus anorganischem Basismaterial, entweder irregulär oder mit sphärischer Geometrie, die einen mittleren Teilchendurchmesser von bevorzugt 0,5 bis 10µm haben.

Besonders geeignet sind Partikel mit einem Durchmesser von 3 - 6µm, da ihre physikalischen Eigenschaften eine schnelle Separation bei optimaler effektiver Bindungsoberfläche gestatten.

Gemäß der vorliegenden Erfindung werden oligomere und/oder polymere Uronsäuren verwendet. Ein Oligomer ist ein Molekül, das aus mehreren strukturell gleichen oder ähnlichen Einheiten aufgebaut ist. Bei einer größeren Anzahl von Einheiten spricht man von einem Polymer. Damit werden bevorzugt Uronsäuren verwendet die mehr als eine monomerische Einheit aufweisen. Bevorzugt stellt der Begriff oligomere und/oder polymere Uronsäuren funktional auf den Zweck der Bindung des biologischen Materials ab. Sofern dies hinreichend möglich ist handelt es ich um eine oligomere und/oder polymere Uronsäure gemäß der vorliegenden Erfindung.

Gemäß einer bevorzugten Ausführungsform werden die Partikel beschrieben, wobei die Partikel magnetisch sind, insbesondere einen magnetischen Kern aufweisen.

Hierdurch kann die Separation der Partikel vereinfacht werden, insbesondere zum Zweck der Automatisierung.

Gemäß einem weiteren Aspekt betrifft die Erfindung die Verwendung der beschriebenen Partikeln zur Anreicherung von biologischem Material, wobei das biologische Material bevorzugt Viren, Bakterien, Apikomplexa, Proteine und insbesondere Nukleinsäuren umfasst. Dabei sind die folgenden Schritte umfasst:
(1) Bereitstellen einer Probe umfassend das biologische Material. Wahlweise kann die Probe chemisch, physikalisch und biologisch oder kombiniert chemisch, physikalisch und biologisch vorbehandelt werden um beispielsweise Komponenten, die eine effektive Bindung der biologischen Materialien an die erfindungsmäßen Partikel erschweren zu beseitigen.
(2) Zugegeben der Partikeln zu der Probe, die gegebenenfalls vorbehandelt wurde, wobei wahlweise Puffersubstanzen oder andere die Bindung der biologischen Materialien an die mit Alginsäuren modifizierten Partikel befördernden Substanzen addiert werden können. Bindungsfördernde Substanzen können unter anderem sein:
   Detergenzien, mit Wasser mischbare Lösungsmittel, Salze einwertiger Kationen, Polyamine sowie Gemische aus diesen Substanzen.
(3) Abtrennen der Partikel mit den gebundenen biologischen Materialien.

Probenmaterial können jede Art von Körperflüssigkeit sein, insbesondere Plasma, Urin, Gehirn- oder Rückenmarkflüssigkeit. Ganz besonders eignet sich als Probenmaterial auch biologisches Material in konservierenden Medien. Beispielhaft seien hier Transportmedien für Viren enthaltene Proben (Abstriche) aufgeführt. Darüber hinaus können als Probenmaterial auch Umweltproben wie Oberflächenwasser, Abwasser oder Grundwasser dienen.

Das Abtrennen kann bei Verwendung von Partikeln, welche magnetisch sind, insbesondere einen magnetischen Kern aufweisen, durch Anlegen externer Magnetfelder erfolgen. Hierdurch wird die Separation der Partikel vereinfacht, insbesondere zum Zweck der Automatisierung.

Gemäß einer bevorzugten Ausführungsform wird die Verwendung beschrieben, wobei die Anreicherung des biologischen Materials im Wesentlichen ohne Zugabe von zwei- oder mehrwertigen Metallkationen erfolgt. Insbesondere wird auf die Zugabe von Kalzium-haltigen Salzen, insbesondere CaCl₂ verzichtet.

Sofern im Zusammenhang der vorliegenden Erfindung von zwei- oder mehrwertigen Kationen die Rede ist, so sind hiermit anorganische Metallkationen mit wenigstens zwei positiven Ladungen am Metallzentrum gemein. Im Falle von CaCl₂, weist das Calcium Kation beispielsweise zwei positive Ladungen auf, und ist damit zweiwertig. Von dieser Definition sind hingegen insbesondere nicht solche organische Kationen umfasst, die zwar mehrere positive Ladungen aufweisen, wobei die positiven Ladungen an mehreren Stellen im Molekül auftreten.

Gemäß einer bevorzugten Ausführungsform wird die Verwendung beschrieben, wobei die Anreicherung des biologischen Materials in Gegenwart von Nichtmetall-Polykationen, insbesondere linearer und verzweigter Polyamine oder linearer oder verzweigter Polyimine erfolgt. Bevorzugt finden Polyethylenimine, besonders bevorzugt Polyethylenimine mit einer Molekülmasse von kleiner 100.000 g/Mol Anwendung. Besonders geeignet sind Polyethylenimine mit einer Molekülmasse unter 20.000 g/Mol, besonders bevorzugt unter 10.000 g/Mol und ganz besonders bevorzugt unter 5.000g/Mol. Es können sowohl lineare, als auch vernetzte Polyethylenimine zu Anwendung kommen, auch solche, die quartärnare Aminogruppen enthalten.

Es wurde überraschend festgestellt, dass hierdurch die Bindung von pathogenen Viren an die Partikel verstärkt wird.

Bereits gering konzentrierte Alginat-Lösungen gelieren beim Einbringen in ausreichend konzentrierten Lösungen von Calciumsalzen schnell. Auch andere divalente oder multivalente Metallkationen können einen ähnlichen Effekt bewirkten wie die Ca²⁺ Ionen. Zu nennen sind insbesondere Erdalkalisalze wie Sr²⁺, Ba²⁺. Aber auch Zn²⁺ oder das dreiwertige Al³⁺. Diese vernetzen die wasserlöslichen monovalenten Salze der Alginsäure unter Hydrogelbildung. Dies ist nachteilig dahingehend, dass eine Automatisierung des Anreicherungsverfahrens erschwert wird da eine Isolation der entstandenen Hydrogele nur unter erheblichem Aufwand möglich ist.

Gemäß einer bevorzugten Ausführungsform wird die Verwendung beschrieben, wobei die Anreicherung des biologischen Materials bei neutralem oder schwach saurem pH-Wert erfolgt, vorzugsweise bei pH-Werten im Bereich der pKs-Werte der Alginsäuren, besonders bei pH Werten zwischen 4 und 6.

Gemäß einer bevorzugten Ausführungsform wird die Verwendung beschrieben, wobei ferner folgende weitere Schritte umfasst sind:
Vollständiges oder teilweises Ablösen des biologischen Materials von der Oberfläche der Partikel;
Verwendung des abgelösten biologischen Materials in einem nachfolgenden Analyseschritt.

Das angereicherte biologische Material kann von den erfindungsgemäßen Oberflächen durch herkömmliche Verfahren, insbesondere Lyse von Zellen, enzymatische oder chemische Zerstörung bestimmter Teile des biologischen Materials abgelöst werden und anschließend einem Analysengang zugeführt werden.

In einer besonderen Ausführungsform kann das an die erfindungsmäßigen Partikel gebundene biologische Material aber auch direkt analysiert werden. D.h. die Partikel mit dem gebundenen biologischen Material direkt in den Analysengang eingespeist werden.

Gemäß einer besonders bevorzugten Ausführungsform betrifft die Anreicherung von biologischem Material Viruspartikel, insbesondere RNA-Viren, wobei diese anschließend mittels einer RT-PCR analysiert werden. Hier ist insbesondere die reverse Transkriptase von Bedeutung, welche zum Nachweis von RNA-Viren verwendet wird.

Eine reverse Transkriptase (RT) ist ein Enzym, das verwendet wird, um komplementäre DNA (cDNA) aus einer RNA-Matrize zu erzeugen, ein Prozess, der als reverse Transkription bezeichnet wird. Reverse Transkriptasen werden von Viren wie HIV verwendet, um ihre Genome zu replizieren, von mobilen genetischen Retrotransposon-Elementen, um sich innerhalb des Wirtsgenoms zu vermehren, und von eukaryotischen Zellen, um die Telomere an den Enden ihrer linearen Chromosomen zu verlängern.

Retrovirale RT hat drei aufeinanderfolgende biochemische Aktivitäten: RNA-abhängige DNA-Polymerase-Aktivität, Ribonuklease H (RNase H) und DNA-abhängige DNA-Polymerase-Aktivität. Zusammen ermöglichen diese Aktivitäten dem Enzym, einzelsträngige RNA in doppelsträngige cDNA umzuwandeln. Bei Retroviren und Retrotransposons kann diese cDNA dann in das Wirtsgenom integriert werden, aus dem durch Wirtszelltranskription neue RNA-Kopien hergestellt werden können. Dieselbe Reaktionsfolge wird im Labor häufig verwendet, um RNA in DNA umzuwandeln, um sie beim molekularen Klonen, der RNA-Sequenzierung, der Polymerase-Kettenreaktion (PCR) oder der Genomanalyse zu verwenden. In dieser Weise können mittels der erfindungsgemäßen Partikel gewonnen RNA-Viren weitergehend analysiert werden, ohne dass es zu einer Inhibition der Transkriptase durch mehrwertige Kationen, insbesondere Calcium, kommt.

Die vorliegende Erfindung ermöglicht vorteilhafterweise die Anreicherung von biologischem Material, wie beispielsweise RNA-Viren, ohne die Verwendung von zwei oder mehrwertigen Metallkationen, die insbesondere eine reverse Transkriptase in einem nachgeschalteten Analyseschritt inhibieren können.

Die erfindungsgemäßen Partikel können daher ganz besonders bevorzugt zur Anreicherung und zum Nachweis von SARS-COV-2 Viren verwendet werden. Hierzu wird als nachgeschalteter Analyseschritt eine RT-PCR eingesetzt, welche nachfolgend beschrieben wird.

Reverse Transkriptions-Polymerase-Kettenreaktion (RT-PCR) ist eine Labortechnik, die die reverse Transkription von RNA in DNA (in diesem Zusammenhang als komplementäre DNA oder cDNA bezeichnet) und die Amplifikation spezifischer DNA-Ziele mithilfe der Polymerase-Kettenreaktion (PCR) kombiniert. Es wird hauptsächlich verwendet, um das Vorliegen und die Menge einer bestimmten RNA zu messen. Dies wird erreicht, indem die Amplifikationsreaktion mithilfe eines Fluoreszenzsignals überwacht wird, einer Technik, die als Echtzeit-PCR oder quantitative PCR (qPCR) bezeichnet wird.

Die Quantifizierung von mRNA mittels RT-PCR kann entweder als einstufige oder als zweistufige Reaktion erfolgen. Der Unterschied zwischen den beiden Ansätzen liegt in der Anzahl der Röhrchen, die bei der Durchführung des Verfahrens verwendet werden. Die zweistufige Reaktion erfordert, dass die Reverse-Transkriptase-Reaktion und die PCR-Amplifikation in getrennten Röhrchen durchgeführt werden. Der Nachteil des zweistufigen Ansatzes ist die Kontaminationsanfälligkeit durch häufigere Probenhandhabung.

Alternativ kann die gesamte Reaktion von der cDNA-Synthese bis zur PCR-Amplifikation in einem einzigen Röhrchen im sogenannten Ein-Schritt-Ansatz stattfinden. Es wird angenommen, dass der Ein-Schritt-Ansatz experimentelle Variationen minimiert, indem alle enzymatischen Reaktionen in einer einzigen Umgebung erfolgen. Dieses Vorgehen eliminiert die Schritte des Pipettierens des cDNA-Produkts in ein neues Gefäß, das arbeitsintensiv und kontaminationsanfällig ist.

Das Genom von SARS-CoV-2 ist über 29,7 kb groß und damit eines der umfangreichsten unter den RNA-Viren. Das Virion von SARS-CoV-2 ist zwischen 50 und 140 Nanometern groß und wird in der Regel im nahen menschlichen Kontakt durch Tröpfchen und Aerosole übertragen. Für die weltweite Ausbreitung spielten besonders größere Übertragungsereignisse, sogenannte Superspreading-Events, eine wichtige Rolle.

Daher ist wegen dessen außerordentlicher weltweiter Bedeutung der effiziente Nachweis von SARS-COV-2 Viren von herausragender Bedeutung und kann mittels der erfindungsgemäßen Partikel erfolgen.

Wie bereits oben beschrieben kann das angereicherte biologische Material von den erfindungsgemäßen Partikeln durch herkömmliche Verfahren, insbesondere Lyse von Zellen, enzymatische oder chemische Zerstörung bestimmter Teile des biologischen Materials abgelöst werden und anschließend einem Analysengang zugeführt werden. In einem besonderen Anwendungsfall können aber auch biologische Materialien die entsprechend der vorliegenden Erfindung angereichert wurden direkt, also wenn sie noch an die erfindungsgemäßen Oberflächen gebunden sind, in einen Analysengang eingespeist werden. Gemäß einer bevorzugten Ausführungsform wird daher die Verwendung beschrieben, wobei die direkte Verwendung der Partikel mit dem biologischen Material in einem nachfolgenden Analyseschritt erfolgt, ohne vorhergehendes Ablösen des biologischen Materials von der Oberfläche der Partikel.

Gemäß einem weiteren Aspekt wird ein Verfahren zur Herstellung der Partikel beschrieben umfassend die Schritte:
Bereitstellen von Partikeln aus einem anorganischen Basismaterial;
Funktionalisieren der Partikel zur Anbindung eines molekularen Linkers (L); und
Binden von oligomeren und/oder Polymeren Uronsäuren an die Partikel unter Nutzung des molekularen Linkers (L).

Funktionalisieren der Partikel zur Anbindung eines molekularen Linkers (L) erfolgt bevorzugt durch Aminoalkylierung von Kieselgel. Das Kieselgel ist bevorzugt ein magnetisches Kieselgel, welches die spätere Abtrennung vereinfacht. Die Aminoalkylierung des Kieselgels ist bevorzugt eine Aminopropylierung, welche sich für die Verlinkung der Alginate als besonders bevorzugt erwiesen hat.

Bevorzugt wird bei der Funktionalisierung Aminopropyl-triethoxy-silan in einem organischen Lösungsmittel gelöst, wobei bevorzugt Acetonitril verwendet wird. Nach wenigstens 12 Stunden, bevorzugt wenigstens 24 Stunden wird das Kieselgel isoliert und dieses gewaschen wobei Acetonitril und/oder Ethanol verwendet wird. Optional wird dieses an Luft getrocknet und im Trockenschrank gehalten.

Die Partikel können nun durch Binden von oligomeren und/oder Polymeren Uronsäuren an die Partikel mittels eines molekularen Linkers weiter funktionalisiert werden.

Hierzu wird das aminoalkylierte Kieselgel in einem geeigneten Puffer aufgenommen. Der Puffer ist mit Alginsäure versetzt. Die festen Bestandteile der Mischung werden bevorzugt abgetrennt. Anschließend wird die Linkersubstanz, welche bevorzugt ein Carbodiimid ist, beispielsweise {1-Ethyl-3-(3-dimethylaminopropyl)-carbodiimid-hydrochlorid} oder auch Dicyclohexylcarbodiimid, DDC, hinzugefügt. Nach einiger Zeit kann die Zielsubstanz gewonnen werden.

Alternativ zu der oben beschriebenen Methode kann auch eine Funktionalisierung mittels Trichlortriazin erfolgen.

Hierzu wird aminoalkyliertes Kieselgel, bevorzugt aminopropyliertes magnetisches Kieselgel, und ein Triazin, bevorzugt Trichlor-triazin, in einem organischen Lösungsmittel, bevorzugt in Toluen, vermischt. Hierzu wird ein Trialkylamin gegeben und die Mischung erhitzt. Nach Abkühlen auf Raumtemperatur wird der Feststoff separiert und der Feststoff optional getrocknet.

Nachfolgend findet ein Binden von Alginsäure an das mit dem Triazinaktivierte aminoalkylierte Kieselgel statt, wobei Alginsäure in Wasser suspendiert und alkalisch gemacht wird. Es wird nachfolgend das aktivierte Kieselgel hinzugegeben. Die festen Bestandteile des Reaktionsgemisches werden abgetrennt, optional gewaschen.

Weitere bevorzugte Ausgestaltungen der Erfindung ergeben sich aus den übrigen, in den Unteransprüchen genannten Merkmalen.

Die verschiedenen in dieser Anmeldung genannten Ausführungsformen der Erfindung sind, sofern im Einzelfall nicht anders ausgeführt, mit Vorteil miteinander kombinierbar.

Die Erfindung wird nachfolgend in Ausführungsbeispielen erläutert.

Die vorliegende Erfindung soll nur anhand von Beispielen dargestellt werden, ohne die Erfindung auf diese Beispiele einzuschränken.

Es wurden die folgenden Beispiele 1 bis 7 beschrieben:
Beispiel 1: Aminopropylierung von magnetischem Kieselgel
Beispiel 2: Binden von Alginsäure auf aminopropyliertes Kieselgel mittels EDCI
Beispiel 3: Aktivieren von aminopropyliertem Kieselgel mit Trichlor-triazin
Beispiel 4: Binden von Alginsäure an mit Trichlortriazin aktiviertem aminopropyliertem Kieselgel
Beispiel 5: Isolation von zellfreier DNA (cfDNA) aus humanem Plasma
Beispiel 6: Anreicherung von viralen Partikeln mit kovalent an Mikropartikel gebundener Alginsäure
Beispiel 7: Anreicherung von Bakterien mit kovalent an Mikropartikel gebundener Alginsäure

Die Beispiele 1 bis 4 beschreiben die Funktionalisierung von Partikeln mit Polyuronsäuren. Die Beispiele 5, 6 und 7 beschreiben die Verwendung der funktionalisierten Partikel zur Anreicherung von biologischem Material. Die Versuchsdurchführungen werden im Folgenden im Einzelnen beschrieben:
Beispiel 1:
   Aminopropylierung von magnetischem Kieselgel
   10g magnetisches Kieselgel (hergestellt nach DE19912799) werden in 100mL 5%iger Lösung von Aminopropyl-triethoxy-silan in Acetonitril (v/v) suspendiert und 48h bei Raumtemperatur belassen. Die Mischung wird gelegentlich aufgeschüttelt. Nach 48h saugt man das Kieselgel ab, wäscht einmal mit Acetonitril und 2x mit Ethanol, trocknet an der Luft und backt das aminopropylierte Produkt 2h bei 125°C in einem Trockenschrank.
Beispiel 2:
   Binden von Alginsäure auf aminopropyliertes Kieselgel mittels EDCI
   2g des unter Beispiel 1 hergestellten aminopropylierten Kieselgels werden in 50mL PBS (Phosphat Buffered Saline), welches 2% (w/v) Alginsäure enthält für 12h auf einer rotierenden Plattform bewegt.

Dann werden die festen Bestandteile der Mischung abgetrennt (z.B. durch Filtration) und mit 50mL PBS gewaschen.

Anschließend nimmt man den Feststoff in 50mL PBS auf und addiert 50mg EDCI {1-Ethyl-3-(3-dimethylaminopropyl)-carbodiimid-hydrochlorid}.

Die Mischung wird über Nacht wiederum auf einer rotierenden Plattform bewegt, anschließend der Feststoff abgetrennt. mit Wasser gewaschen und in einem geeigneten Puffersystem (z.B. 10mM TrisHCl, pH 7, 1mM EDTA) oder Wasser suspendiert.

### Beispiel 3:

Aktivieren von aminopropyliertem Kieselgel mit Trichlor-triazin
5g aminopropyliertes magnetisches Kieselgel und 1,5g Chlortriazin werden mit 50mL Toluen vermischt. Dann gibt man 1mL Trieethylamin zu und erhitzt im Ölbad auf 90°C (Badtemperatur) und inkubiert 3h bei dieser Temperatur.

Nach Abkühlen auf Raumtemperatur wird weiter für 12h (über Nacht) gerührt.

Der Feststoff wird mittels eines Magneten separiert, die Flüssigkeit abdekantiert und die feste Phase 3x mit 50mL Toluen gewaschen.

Dann saugt man den Feststoff ab, wäscht noch einmal mit Toluen und trocknet an der Luft

### Beispiel 4:

Binden von Alginsäure an mit Trichlortriazin aktiviertem aminopropyliertem Kieselgel
500mg Alginsäure (Roth) werden in 25mL Wasser suspendiert und mit 5mL 1M NaOH versetzt. Es wird gewartet, bis sich die Alginsäure komplett aufgelöst hat..

Dann addiert man 1g des unter Beispiel 3 hergestellten aktivierten magnetischen Kieselgels und bewegt die Mischung über Nacht.

Die festen Bestandteile des Reaktionsgemisches werden abgetrennt, mit Wasser gewaschen und in einem geeigneten Puffersystem aufgenommen (z.B. 10mM TrisHCl, pH 7; 1mM EDTA).

### Beispiel 5:

Isolation von zellfreier DNA (cfDNA) aus humanem Plasma
Zu 4mL humanem Plasma (mit Citrat als Antikoagulant) werden 100µL einer Proteinase K Lösung (20mg Proteinase K/mL Wasser) gegeben, gemischt und bei 60°C 30min lang inkubiert. Nach Abkühlen des lysierten Plasmas addiert man 2mg des im Beispiel 2 hergestellten mit Alginsäure modifizierten Kieselgels, mischt und bewegt die Mischung 10min.

Dann werden die magnetischen Partikel mit Hilfe eines Permanentmagneten separiert, der Überstand verworfen und auf die Partikel mit der angereicherten zellfreien DNA 300µL einer Lösung von 6M Guanidinthiocyanat in Wasser gegeben. Hierdurch löst sich die angereicherte zellfreie DNA von den Anreicherungspartikeln und kann nach bekannten Verfahren isolierte werden. Z.B. unter Verwendung des LGC magMini Kits (Kat.Nr.: NAP40401).

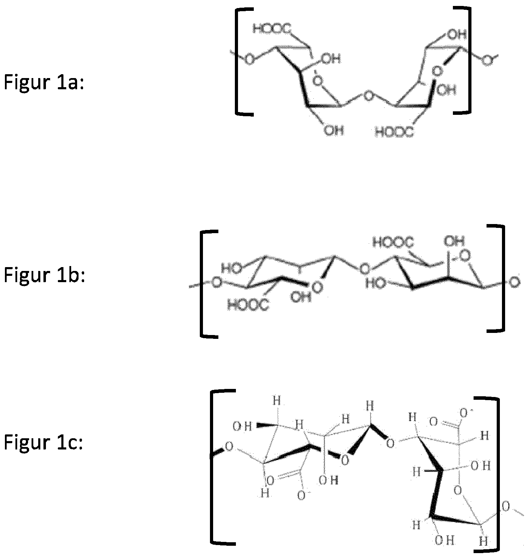

Abbildung 1 zeigt eine analytische Elektrophorese der isolierten zellfreien DNA auf einem AGILENT BioAnalyser (highSensitivity - Chip)

### Beispiel 6:

Anreicherung von viralen Partikeln mit kovalent an Mikropartikel gebundener Alginsäure Zu 2mL einer Probe, die ca. 500 Genomäquivalente SARS Cov 2/mL Flüssigkeit enthält (z.B. in PBS) werden 10mg der unter Beispiel 2 hergestellten mit Alginsäure gebundenen magnetischen Mikropartikel gegeben. Man bewegt die Mischung und separiert anschließend die Magnetpartikel mit den gebundenen viralen Partikeln.

Die virale Nukleinsäure kann im Anschluss an die Anreicherung mit Hilfe des Sbeadex-Pathogen Nucleic Acid Purification Kits (LGC Biosearch Technologies, Kat.Nr.: NAP40-024-02) isoliert und mittels RT-qPCR nachgewiesen werden.

Der Nachweis über Nukleinsäure Amplifikation erfolgt hierbei mit dem Promega GoTaq^{®} 1-Step RT-qPCR System (ref. A6121) und Biosearch Technologies 2019-nCoV CDC Probe and Primer Kit for SARS-CoV-2.

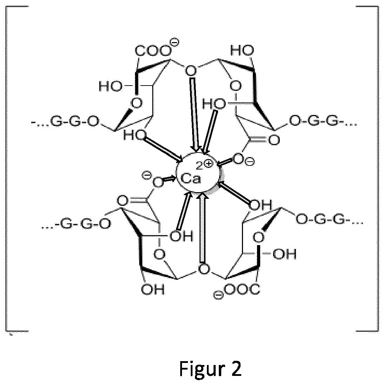

Die entsprechenden Amplifikationsdiagramme, die mittels des 7500 Real Time PCR - Systems (Fa. Applied Biosystems) erzielt wurden sind in Abbildung 2 exemplarisch dargestellt.

### Beispiel 7:

Anreicherung von Bakterien mit kovalent an Mikropartikel gebundener Alginsäure
4mL einer Bakteriensuspension die variable Mengen der Spezies Bordetalla pertussis, Chlamydophila pneumoniae, Coxiella burnetii und Chlamydophila psitacci enthält (z.B. hergestellt aus dem Standard AmpliRun^{®} -Vircell S.L. Artikel Nr. MBTC020), werden mit 10mg der Beispiel 2 hergestellten magnetischen Kieselgelpartikel, an die Alginsäuren kovalent fixiert sind, gemischt und 90µL Essigsäure zugegeben.

Diese Mischung wird 10min konstant in Bewegung gehalten. Anschließend trennt man die Beads mit den angereicherten Bakterien ab und behandelt das Beadpellet mit 150ul der QuickextractTM Lösung entsprechend dem Protokoll des QuickextractTM-Kits der LGC Biosearch (Kat.Nr.: QE09050).

Die entstandene Lösung enthält die bakteriellen Nukleinsäuren und kann direkt als Template-Lösung in eine PCR eingesetzt werden.

Der Nachweis bakterieller DNA erfolgt im vorliegenden Beispiel mit Hilfe von Angaben aus der Literatur [Grogan, J.A., et al. (2011). J. Med Microbiol, 60:722-729; Hardick, J. et al. (2004). J Mol Diagn, 6(2):132-136; De Bruin, A., et al. (2011). Appl Environ Microbiol, 77(18):6516-6523; Menard, A., et al. (2006). J Med Microbiol. 55(4): 471-473]

Die Realtime PCR erfolgte mittels des 7500 Real Time PCR Systems, (Fa. Applied Biosystems). Die Analyse erfolgte anhand von zellfreier DNA, angereichert aus 4mL humanem Plasma, an magnetischen Mikropartikeln, die mit linearen Alginsäuren modifiziert sind. Die Analyse erfolgte mit dem HS-Chip auf dem BioAnalyzer (Fa. Agilent)

Ferner wurde eine PCR Amplifikation von SARS CoV-2 Nukleinsäure nach Anreicherung mit den erfindungsgemäßen Partikeln und RNA-Extraktion mit dem Sbeadex-Pathogen Kit erfolgreich durchgeführt.

Ferner wurden eine PCR-Amplifikation von bakterieller DNA nach Bakterienanreicherung und DNA-Isolation mit den erfindungsgemäßen Partikeln erfolgreich durchgeführt.

Die Erfindung wird im Folgenden anhand der Abbildungen beschrieben. Es zeigen:
- Figur 1a:: lineare 1-4 glycosidische verknüpfte Einheiten aus α-1,4-L-Guluronsäure.
- Figur 1b:: lineare 1-4 glycosidische verknüpfte Einheiten aus β-1,4-D-Mannuronsäure.
- Figur 1c:: lineare 1-4 glycosidische verknüpfte Einheiten aus α-1,4-L-Guluronsäure /β-1,4-D-Mannuronsäure.
- Figur 2:: Ca²⁺-Alginsäure Komplex als molekularer Baustein des Alginsäurehydrogels gemäß dem Stand der Technik.
- Figur 3:: Mit oligomeren und/oder polymeren Uronsäuren mittels eines molekularen Linkers (L) funktionalisierte Partikel gemäß der Erfindung.
- Figur 4:: Reaktionsschema zum Binden von Carboxylsäure Funktionalitäten der Uronsäuren an amino-aktivierte Partikel mittels Carbodiimid.

### Im Folgenden werden die Figuren im Detail beschrieben

Die Figuren 1a, 1b und 1c zeigen Alginatstrukturen, wie diese gemäß der vorliegenden Erfindung zur Funktionalisierung von Partikeln genutzt werden können. Alginate sind Polysaccharide, die aus zwei 1,4-verknüpften Uronsäurebausteinen aufgebaut sind, wobei es sich um α-D-Mannuronsäure und der α-L-Guluronsäure handelt.

Figur 1a zeigt lineare 1-4 glycosidisch verknüpfte Einheiten aus α-1,4-L-Guluronsäure, während Figur 1b lineare 1-4 glycosidisch verknüpfte Einheiten aus β-1,4-D-Mannuronsäure zeigt. In Figur 1c werden hingegen lineare 1-4 glycosidisch verknüpfte Einheiten aus α-1,4-L-Guluronsäure und β-1,4-D-Mannuronsäure gezeigt. Alginsäuren weisen diese Substrukturtypen von linearen 1-4 glycosidische verknüpften Einheiten auf. Auch Quervernetzungen sind möglich.

Homopolymere Bereiche, in denen Mannuronsäure oder Guluronsäure jeweils als Block vorliegen, werden als G- oder M- Blöcke bezeichnet. In den Abbildungen sind diese als Figur 1a und Figure 1b gezeigt. Diese wechseln sich mitunter mit heterogenen Abschnitten ab, in denen die beiden Bausteine alternierend auftreten. Diese werden als GM-Blöcke bezeichnet, was in siehe Figur 1c gezeigt ist.

Die Alginate werden aus Braunalgen gewonnen, in denen sie interzellular als strukturgebendes Element im Zellwandbereich vorkommen. Aus der Stereochemie und der Verknüpfung der beiden Uronsäuren ergibt sich teils eine Kettenkonformation, welche vorwiegende durch die M-Blöcke gebildet ist, wie diese in Figur 1b gezeigt sind. Teils wird jedoch eine zickzackartige Konformation durch die G-Blöcke gebildet, wie diese in Figur 1a gezeigt sind. Die alternierenden GM-Bereiche gemäß Figur 1c hingegen können als treppenartig angeordnet beschrieben werden.

Figur 2 zeigt nun einen Ca²⁺-Alginsäure Komplex als molekularen Baustein des Alginsäurehydrogels. Die unter Figur 1 beschriebenen Kettenkonformation ist essentiell für die Art der Gelbildung mit Calciumionen, die in die "Taschen" der G- Blöcke passen und dort mit mehreren Sauerstoffen in geeigneter räumlicher Anordnung koordinieren. So können zwei oder mehr Polymerketten physikalisch miteinander vernetzt werden. Ebenso wichtig sind M-Blöcke, die frei beweglichen Verbindungen zwischen den G-Zonen darstellen. Es kommt so zur Ausbildung eines dreidimensionalen Netzwerks, in das Wasser eingelagert wird. Der osmotische Druck der wässrigen Phase und der mechanische Gegendruck des Netzwerks bestimmen den Quellungszustand. Die Calciumionen befinden sich in den Hohlräumen der Zickzack-Strukturen. Die Ausbildung eines stabilen dreidimensionalen Netzwerks über physikalische Wechselwirkungen erfordert das kooperative Zusammenwirken mehrerer solcher schwacher Wechselwirkungen. Man geht davon aus, dass etwa 6-10 Wechselwirkungspunkte pro Verbindungszone wirksam sind. Die Stabilität der Alginatgele und ihre Festigkeit hängen also auch von den Blocklängen der GG-, MM- und GM-Sequenzen ab. Ein anschauliches Modell für die Kooperativität stellt der Klettverschluss dar, bei dem auch nur das Zusammenspiel vieler kleiner Verhakungen zu einer stabilen Verbindung führt.

Neben einer Vernetzung durch Zugaben von zwei-wertigen oder mehr-wertigen Kationen kann die Zugaben von Mineralsäure zu einem Gelieren der Alginsäuren führen. Säuert man Alginate an, drängt man die Dissoziation der Uronsäuren zurück. Es bildet sich neutrale Alginsäure, die nicht mehr wasserlöslich ist.

Die vorliegende Erfindung hingegen benutzt chemische Methoden um das Alginat, welches Carboxylgruppen enthält, kovalent an aminopropylierte Partikel zu binden.

So bleiben die Eigenschaften von nicht geliertem Alginat erhalten. Diese ist im Kontrast zu den Lösungen aus dem Stand der Technik zu sehen, bei welchen ein vollständige Gelbildung mit relativ unspezifischen und damit ungenauen Wechselwirkungen wirksam wird.

Die vorliegende Erfindung hingegen beschreibt die Fähigkeit eine Komplexbildung an zellfreie DNA oder Partikel, wie Viruspartikel, herbeizuführen, und zwar ohne Zusatz von Mineralsäuren oder Metallkationen. Gemäß der Erfindung wird damit auf eine vollständige Gelbildung verzichtet, was spezifische und damit genaue Wechselwirkungen mit den zu isolierenden Zielstrukturen ermöglicht.

Ferner weisen die aus dem Stand der Technik erforderlichen Zusätze von mehrwertigen Metallkationen sich dahingehend negativ aus, dass diese inhibierend auf Enzyme wirken können die in nachgeschalteten Verfahren verwendet werden, insbesondere in Analyseverfahren.

Hier ist insbesondere die reverse Transkriptase zu nennen, welche bei Nachweis beispielsweise von RNA-Viren verwendet wird. Auch wirken zweiwertige und/oder mehrwertige Metallkationen inhibierend auf andere Downstream-Applikationen.

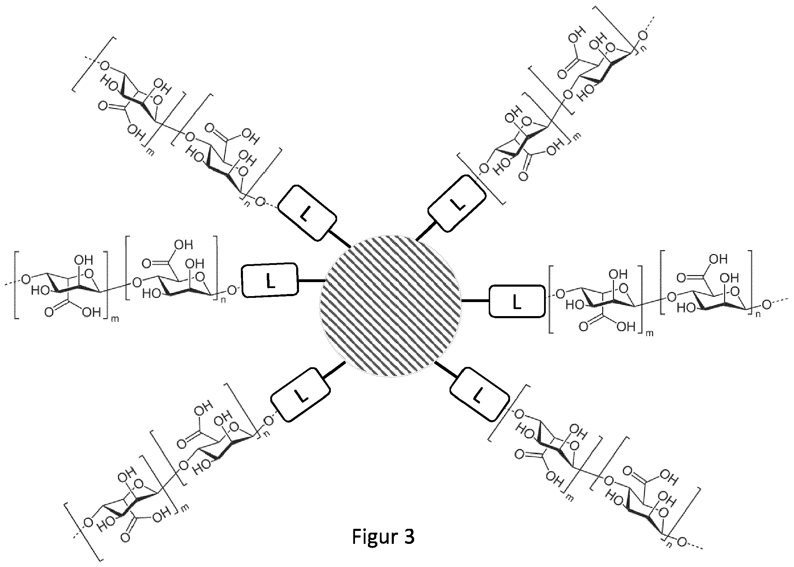

Figur 3 zeigt die erfindungsgemäßen Partikel, welche mit oligomeren und/oder polymeren Uronsäuren mittels eines molekularen Linkers (L) funktionalisiert wurden. Bei den Methoden aus dem Stand der Technik ist nachteilig, dass ein Alginat-DNA-Partikel Komplex "zufällig" entsteht, während bei der erfindungsgemäßen Methode die gezeigten funktionalisierten Partikel verwendet werden, welche in der Lage sind über spezifische Wechselwirkungen diverse Partikel oder DNA zu binden. In der Figur sind aus Darstellungsgründen nur Modifikationen gezeigt, welche eine endständige Oberflächenverknüpfung ausbilden. Grundsätzlich weisen die oligomeren und/oder polymeren Uronsäuren aber an allen Struktureinheiten funktionelle Gruppen auf, mit welchen diese an die Oberfläche gebunden werden können. Die vorliegende Erfindung ist also nicht auf endständige Oberflächenverknüpfung beschränkt.

Figur 4 zeigt ein Reaktionsschema zum Binden von Carboxylsäure Funktionalitäten der Uronsäuren an amino-aktivierte Partikel mittels Carbodiimid. Durch die Verwendung eines Carbodiimid Kopplungsreagenz wird die Polyuronsäure über die Carboxylgruppe vorübergehend in eine reaktive Zwischenstufe überführt, die anschließend bereitwillig mit einem Nukleophil reagiert. Im vorliegenden Fall stellt das Nukleophil eine Aminogruppe dar welche durch Aktivierung der Oberfläche der Partikel eingeführt wird. Grundsätzlich können eine Vielzahl an Kupplungsreagenzien verwendet werden. Da nicht jede Kombination der aktivierten Partikel mit einem Kupplungsreagenz die gleiche Ausbeute aufweist, sind mache Verfahren beziehungsweise Reagenzien bevorzugt. Wasserlösliche Kupplungsreagenzien können zur Immobilisierung der Polyuronsäuren in wässrigen Lösungen verwendet werden.

## Patentansprüche

1. Partikel zur Anreicherung von biologischem Material, **dadurch gekennzeichnet, dass** oligomere und/oder polymere Uronsäuren an die Partikel gebunden sind.

2. Die Partikel gemäß dem vorhergehenden Anspruch, wobei die oligomeren und/oder polymeren Uronsäuren lineare 1-4 glycosidische verknüpfte Einheiten aus α-1,4-L-Guluronsäure und/oder aus β-1,4-D-Mannuronsäure aufweisen.

3. Die Partikel gemäß dem vorhergehenden Anspruch, wobei die oligomeren und/oder polymeren Uronsäuren Alginsäuren aus Braunalgen umfassen.

4. Die Partikel gemäß einem der vorhergehenden Ansprüche, wobei das biologische Material Viren, Bakterien, Apikomplexa, Proteine und insbesondere Nukleinsäuren umfasst.

5. Die Partikel gemäß einem der vorhergehenden Ansprüche, wobei die oligomeren und/oder polymeren Uronsäuren mittels eines molekularen Linkers (L) an die Partikel gebunden sind.

6. Die Partikel gemäß einem der vorhergehenden Ansprüche, wobei die Uronsäuren durch Umsetzung mit einem Carbodiimid an den molekularen Linker gebunden werden.

7. Die Partikel gemäß einem der vorhergehenden Ansprüche, wobei die Partikel ein anorganisches Basismaterial umfassen, wobei das anorganische Basismaterial bevorzugt Siliziumoxid ist.

8. Die Partikel gemäß einem der vorhergehenden Ansprüche, wobei die Partikel magnetisch sind, insbesondere einen magnetischen Kern aufweisen.

9. Verwendung von Partikeln gemäß einem der vorhergehenden Ansprüche zur
Anreicherung von biologischem Material, wobei das biologische Material Viren, Bakterien, Apikomplexa, Proteine und insbesondere Nukleinsäuren umfasst, und wobei
die folgenden Schritte umfasst sind:
µ Bereitstellen einer Probe umfassend das biologische Material und wenn erforderlich eine chemische, physikalische oder biologische Vorbehandlung der Probe, insbesondere eine kombinierte chemische, physikalische und biologische Vorbehandlung der Probe ;
• Zugegeben der Partikeln zu der Probe und Inkubation des Gemisches; und
• Abtrennen der Partikel.

10. Verwendung gemäß den vorhergehenden Ansprüchen, wobei
die Anreicherung des biologischen Materials unter Zugabe von Polykationen erfolgt,
insbesondere linearer und verzweigter Polyamine oder linearer oder verzweigter Polyimine erfolgt, insbesondere unter Verwendung von Polyethylenimin.

11. Verwendung gemäß den vorhergehenden Ansprüchen, wobei
die Anreicherung des biologischen Materials im Wesentlichen ohne Zugabe von zwei- oder mehrwertigen Metallkationen erfolgt und/oder
die Anreicherung des biologischen Materials bei einem pH-Wert zwischen 3 und 7, insbesondere bei einem pH Wert zwischen 4 und 5 erfolgt.

12. Verwendung gemäß einem der vorhergehenden Ansprüche, wobei folgende weitere Schritte
umfasst sind:
Ablösen des biologischen Material von der Oberfläche der Partikel;
Verwendung des abgelösten biologischen Materials in einem nachfolgenden Analyseschritt

13. Verwendung gemäß einem der vorhergehenden Ansprüche, wobei
die direkte Verwendung der Partikel mit dem biologischen Material in einem nachfolgenden Analyseschritt ohne vorhergehendes Ablösen des biologischen Material von der Oberfläche der Partikel.

14. Verfahren zu Herstellung der magnetischen Partikel gemäß einem der vorhergehenden Ansprüche, wobei das Verfahren die Schritte umfasst:
Bereitstellen von Partikeln aus einem anorganischen Basismaterial;
Funktionalisieren der Partikel zur Anbindung eines molekularen Linkers (L); und
Binden von oligomeren und/oder polymeren Uronsäuren an die Partikel mittels des molekularen Linkers (L).
